# EUROPÄISCHE PATENTSCHRIFT

(11) **EP 2 164 531 B1**
(45) Veröffentlichungstag und Bekanntmachung des Hinweises auf die Patenterteilung: **20.02.2013**
(21) Anmeldenummer: 08773841.5
(22) Anmeldetag: 03.07.2008
(51) Int. Cl.: A61L 9/12, A61L 9/04, B60H 3/00

(54) **BEDUFTUNGSVORRICHTUNG, INSBESONDERE FÜR EIN KRAFTFAHRZEUG**
AIR FRESHENER DEVICE IN PARTICULAR FOR A MOTOR VEHICLE
DIFFUSEUR DE PARFUM DESTINÉ EN PARTICULIER À UN VÉHICULE À MOTEUR

(30) Priorität: 03.07.2007 DE 102007030943
(43) Veröffentlichungstag der Anmeldung: 24.03.2010
(73) Patentinhaber: Behr GmbH & Co. KG, 70469 Stuttgart (DE)
(72) Erfinder: BROLY, Fabien, F-68320 Bischwihr (FR); SCHMITZ, Jochen, 51381 Leverkusen (DE); BOSCHERT, Björn, 76228 Karlsruhe (DE); D'ANGELO, Marco, 70469 Stuttgart (DE); FIEGER, Martin, 71636 Ludwigsburg (DE); TIDELSKI, Axel, 70435 Stuttgart (DE); ZELLER, Kuno, 70435 Stuttgart (DE)
(74) Vertreter: Grauel, Andreas
(86) Internationale Anmeldenummer: PCT/EP2008/005431
(87) Internationale Veröffentlichungsnummer: WO 2009/003704

(56) Entgegenhaltungen:
- WO-A-00/12143
- WO-A-2004/024483
- WO-A-2006/087610
- GB-A- 2 322 554
- GB-A- 2 401 047
- JP-A- 2002 218 893
- US-A- 4 523 870
- US-A- 5 050 798
- US-B1- 6 511 531

## Beschreibung

Die Erfindung betrifft eine Beduftungsvorrichtung gemäß dem Oberbegriff des Anspruches 1.

Aus der DE 103 27 122 A1 ist eine Beduftungsvorrichtung, insbesondere zum Beduften eines Kraftfahrzeugs, bekannt, welche einen Duftstoff enthält, mit einer Dosiereinrichtung, wobei die Beduftungsvorrichtung zumindest einen Hohlkörper, der mit mindestens einer Öffnung versehen ist, und ein Element, insbesondere einen Hohlkörper, mit mindestens einer weiteren Öffnung umfasst. Dabei ist der Hohlkörper und/oder das Element mittels eines Motors derart antreibbar, dass eine Relativbewegung zwischen dem Hohlkörper und dem Element möglich ist, so dass die beiden Öffnungen in zumindest einer Stellung der Relativbewegung ein Austreten von Duftstoff von innen nach außen ermöglichen. Der Duftstoff selbst ist innerhalb des Hohlkörpers in einer Duftstoffpatrone oder -kartusche angeordnet, die bei Bedarf auswechselbar ist.

Eine andere Beduftungsvorrichtung ist aus der US 5,314,669 A bekannt. Bei dieser Beduftungsvorrichtung sind zwei in axialer Richtung zueinander angeordnete Hohlzylinder in einem im Wesentlichen als Hohlzylinder ausgebildeten Gehäuse drehbar aufgenommen. Der das Gehäuse bildende Hohlzylinder weist zwei rechteckförmige, sich in Längsrichtung erstreckenden Öffnungen in seiner Mantelfläche auf einander gegenüberliegenden Seiten auf, die sich jeweils zumindest über einen Teilbereich der inneren Hohlzylinder erstrecken. Die inneren Hohlzylinder weisen ebenfalls jeweils zwei einander gegenüberliegende Öffnungen in ihren Mantelflächen auf, die jedoch gestuft ausgebildet sind, d.h. die Form dreier jeweils um die Hälfte ihrer Länge versetzt zueinander angeordneter, langgestreckter .und zusammen eine gemeinsame Öffnung bildender Rechtecke aufweisen. Die Öffnungen der inneren Hohlzylinder sind verdreht zueinander angeordnet, so dass verschiedene Beduftungsvariationen möglich sind.

Die WO 00/12143, die US 6511831, die GB 2322554, die WO 2004/024483, die WO 2006/087610 A1 und die US 5050798 offenbaren Beduftungsvorrichtungen nach dem Stand der Technik.

Die US 5,178,327 A offenbart ein Beduftungssystem mit einem in mehrere Sektoren unterteilten, drehbar in einem Gehäuse angeordneten Hohlzylinder, wobei mindestens ein kuchenartiger Teil des Hohlzylinders in der Größe eines Segments fehlt, weshalb auf den Hohlzylinder im Folgenden als Teil-Hohlzylinder Bezug genommen wird. Die Segmente sind jeweils stirnseitig offen und in jedem der Segmente ist ein anderer Duftstoff eingelagert. Im Gehäuse ist auf einer oder beiden Seiten in axialer Richtung des Teil-Hohlzylinders eine in Ihrer Gestalt im Wesentlichen dem Querschnitt eines Segments entsprechende Öffnung ausgebildet. Um einen Raum zu beduften, wird das den gewünschten Duft enthaltende Segment vor die Öffnung gedreht. Wird keine Beduftung gewünscht, so wird das fehlende Segment vor die Öffnung gedreht. Um eine stärkere Beduftung eines Raumes zu ermöglichen, kann auch ein Lüfter vorgesehen sein, welches Luft durch die erste Öffnung in das Gehäuse und nach dem Durchströmen des Teil-Hohlzylinders durch die zweite, gegenüberliegende Öffnung dem zu beduftenden Raum zuführt.

Derartige Beduftungsvorrichtungen/-systeme lassen noch Wünsche offen.

Es ist Aufgabe der Erfindung, eine verbesserte Beduftungsvorrichtung zur Verfügung zu stellen.

Diese Aufgabe wird gelöst durch eine Beduftungsvorrichtung mit den Merkmalen des Anspruchs 1. Vorteilhafte Ausgestaltungen sind Gegenstand der Unteransprüche.

Hierbei ist eine Beduftungsvorrichtung, insbesondere für ein Kraftfahrzeug, vorgesehen, mit einem Gehäuse, zumindest einer in einer im Gehäuse vorgesehenen Aufnahme angeordneten Patrone, in welcher ein Duftstoff enthalten ist, einer Eintrittsöffnung, über welche Luft in das Gehäuse der Beduftungsvorrichtung gelangen kann, einem Mischraum, in welchem der Duftstoff mit der Luft vermischbar ist, einer Austrittsöffnung, über welche die mit dem Duftstoff vermischte Luft nach außen gelangen kann, und mindestens einer betätigbaren Absperrvorrichtung, welche den Duftstoff an einem Austreten aus der Aufnahme in den Mischraum in mindestens einer Stellung hindert, wobei im Bereich der Eintrittsöffnung und/oder zwischen der Eintrittsöffnung und dem Mischraum eine Drallerzeugungsvorrichtung vorgesehen ist.

Eine derartige Beduftungsvorrichtung ist vorzugsweise im Bereich eines Armaturenbretts eines Fahrzeugs angeordnet, insbesondere bevorzugt benachbart einem Luftausströmer. Beim Luftausströmer kann es sich beispielsweise um die Mitteldüse handeln, so dass der Duftstoff zentral im Frontbereich des Fahrzeugs in den Fahrzeuginnenraum gelangt und sich von dort aus verteilt. Insbesondere bevorzugt ist die Beduftungsvorrichtung mit dem zum Luftausströmer führenden Luftkanal über die Eintrittsöffnung verbunden, so dass Luft vom Luftkanal direkt in die Beduftungsvorrichtung einströmen kann und hier mit dem entsprechenden Duftstoff versetzt werden kann, bevor sie die Beduftungsvorrichtung über die Austrittsöffnung wieder verlässt. Die Austrittsöffnung ist hierbei bevorzugt getrennt vom Luftausströmer ausgebildet, jedoch in dessen Nachbarschaft angeordnet. Vorteilhafterweise kann auf die Ausbildung eines Luftkanals zur Eintrittsöffnung verzichtet werden. Über die Eintrittsöffnung kann die Luft dann direkt aus der Umgebung des Einbauraums, beispielsweise einem Bereich innerhalb des Armaturenbretts in die Beduftungsvorrichtung eintreten.

Bei der Drallerzeugungsvorrichtung handelt es sich vorzugsweise um ein drehbares Laufrad und/oder starre Leitschaufeln. Diese sorgen für ein drallbehaftetes Eintreten der Luft in den Mischraum der Beduftungsvorrichtung, so dass der Duftstoff schnell mit der eintretenden Luft vermischt wird. Ist ein Laufrad vorgesehen, so ist dasselbe besonders bevorzugt freidrehend gelagert, wobei als Antrieb die einströmende Luft dient, d.h. es ist als eine Art Luftrad ausgebildet. Das Laufrad kann auch angetrieben werden, bspw. mittels eines Elektromotors. Insbesondere für den Fall, dass die Luft ohne einen gesonderten Luftkanal der Eintrittsöffnung der Beduftungsvorrichtung zugeführt wird ist ein elektromotorisch betriebener Lüfter vorteilhaft um Luft aus der Umgebung des Einbauorts anzusaugen und weiterzubefördern.

Für einen besonders einfachen Patronenwechsel zum Austausch des oder der vorrätigen Duftstoffe oder zum Ersetzen einer leeren Patrone, ist die Aufnahme für die Patrone oder Patronen besonders bevorzugt schubladenförmig ausgebildet. Insbesondere bevorzugt ist die Aufnahme federvorgespannt, so dass sie sich nach einem Drücken auf die Frontseite der Aufnahme selbsttätig öffnet und die Patrone(n) zugänglich macht.

Vorteilhafterweise sind am Gehäuse eine oder mehrere Dichtlippen angeordnet sind, die an einer Fläche eine in die Aufnahme eingesetzten Patrone zur Abdichtung anliegen. Der Raum zwischen der Oberseite der Patrone und dem darüber befindlichen Gehäuse kann insbesondere im Zusammenwirken mit der Absperrvorrichtung hermetisch verschlossen werden. Alternativ oder zusätzlich können entsprechende Dichtlippen auch an der Aufnahme vorgesehen sein, welche dann abdichtend am Gehäuse anliegen.

Die Aufnahme für die Patrone oder Patronen ist vorzugsweise auf der der Drallerzeugungsvorrichtung gegenüberliegenden Seite des Mischraums angeordnet. Dies ermöglicht eine gute Raumausnutzung des vorhandenen Bauraums in einem Armaturenbrett, auch unter Berücksichtigung der Lufteinleitung und Luftausleitung.

Die Absperrvorrichtung, welche ein ungewolltes Austreten von Duftstoff verhindern soll, ist bevorzugt zwischen der Aufnahme und dem Mischraum angeordnet. Eine Absperrvorrichtung an der Austrittsöffnung kann so entfallen.

Die Absperrvorrichtung ist vorzugsweise durch eine Klappe gebildet, wobei die Schwenkachse der Klappe bevorzugt parallel zur Einschubrichtung der Aufnahme verläuft.

Alternativ kann die Absperrvorrichtung auch durch beliebige andere Elemente gebildet sein, beispielsweise durch zwei relativ zueinander verschiebbare, mit mindestens je einer Öffnung versehene Elemente, insbesondere mit einer Mehrzahl von Öffnungen versehene Elemente. Die Betätigung kann beispielsweise mittels eines Hubmagneten erfolgen. Ebenfalls kann die Absperrvorrichtung durch eine sogenannte Jalousieklappe gebildet sein.

Einen weiteren Gegenstand bildet eine Patrone oder Kartusche zur Freigabe eines Duftstoffes, insbesondere zur Verwendung in einer Beduftungsvorrichtung. Die Patrone weist ein Bodenteil, ein Abdeckteil und das Boden- und Abdeckteil verbindende Seitenwände auf, die einen Raum umschließen in dem ein Duftstoff oder ein Duftstoffträger mit einem Duftstoff aufgenommen ist, wobei in dem Abdeckteil eine oder mehrere Öffnungen vorgesehen sind, durch die der Duftstoff austreten kann. Vorteilhafterweise sind die Öffnungen schlitz-, kreis- oder S-förmig ausgebildet.

Bevorzugt ist die Gesamtfläche der Öffnungen im Abdeckteil kleiner als 80%, bevorzugt kleiner als 70%, besonders bevorzugt kleiner als 60% insbesondere kleiner als 50% der Fläche des Abdeckteils, wobei ein Wert von 20% - 30% nicht unterschritten werden soll. Damit wird für den Fall, dass der Patronenaufnahmeschacht in einer Beduftungsvorrichtung unbeabsichtigt längere Zeit geöffnet ist, ein zu großer Austritt von Duftstoff vermieden.

In einer weiteren vorteilhaften Ausgestaltung der Patrone, sind am Abdeckteil eine oder mehrere Dichtlippen angeordnet, insbesondere angespritzt. Bevorzugt verläuft eine Dichtlippe am Randbereich des Abdeckteils umlaufend und die Öffnungen im Abdeckteil einschließend. Beim Einsatz in einer Beduftungsvorrichtung, kann bei einer entsprechenden Anlage an einem Gehäusebereich der Vorrichtung eine luftdichte Verbindung erreicht werden.

In weiterer vorteilhafter Ausgestaltung sind zumindest zwei gegenüberliegende Seitenwände der Patrone nicht genau parallel verlaufend ausgebildet und weisen insbesondere eine Abweichung der Parallelität von insgesamt 0,5 bis 3 mm, bevorzugt von 0,5 mm bis 2 mm auf. Über die Länge der gegenüberliegenden Seitenwände betrachtet, bedeutet dies, dass ein entsprechender Normalabstand der Seitenwände eine Schwankung von dem genannten Ausmaß aufweist, wobei vorzugsweise der Abstand der Seitenwände zueinander, in Längsrichtung betrachtet, in der Mitte einen Maximalwert und an den beiden Endbereichen einen Minimalwert erreicht. So entsteht ein ausgebauchtes Profil der Patrone, welches an den Enden einen Einsatz in einen Aufnahmeschacht einer Beduftungsvorrichtung erleichtert und mittig eine klemmende Fixierung ermöglicht. Der Aufnahmeschacht weist hierzu vorzugsweise parallele Aufnahmewände auf, deren Normalabstand geringer ist als der Maximalwert des Abstandes der beiden Seitenwände, so dass ein entsprechender Klemmsitz für die Patrone gebildet wird. Alternativ oder zusätzlich können an den Seitenflächen auch mehrere Noppen vorgesehen sein um einen Klemmsitz in der Aufnahme zu verbessern.

Bevorzugt ist zumindest eine der Seitenwände abnehmbar oder am Abdeck- oder Bodenteil schwenkbar ausgebildet. Für den Fall einer schwenkbar angeordneten Seitenwand ist insbesondere eine einstückige Verbindung über ein Filmscharnier mit dem Abdeck- oder Bodenteil vorgesehen. Mittels Abnehmens oder Verschwenkens der entsprechenden Seitenwand lässt sich ein einfacher Wechsel bzw. eine Erneuerung des Duftstoffes oder des Duftstoffträgers vornehmen. Alternativ kann auch das Abdeck- oder Bodenteil abnehm- oder schwenkbar ausgebildet sein und die entsprechende Funktion für das Wechseln des Duftstoffes erfüllen.

In einer Lager- oder Transportposition, also in einem Zustand, in welchem sich die Patrone nicht in eine Beduftungsvorrichtung eingesetzt befindet, ist die Patrone mittels eines Deckels, der einen hakenförmigen Rand aufweist und in entsprechende Vorsprünge am Abdeckteil eingreift, verschlossen. Alternativ kann der Verschluss auch über eine Abreißfolie gebildet sein. Der Verschluss mittels eines Deckels hat dabei den Vorteil, dass die Patrone nach bestimmungsgemäßen Gebrauch für eine Zwischenlagerung wiederverschließbar ist.

Im Folgenden wird die Erfindung anhand eines Ausführungsbeispiels mit Varianten, teilweise unter Bezugnahme auf die Zeichnung, im Einzelnen erläutert. Es zeigen:

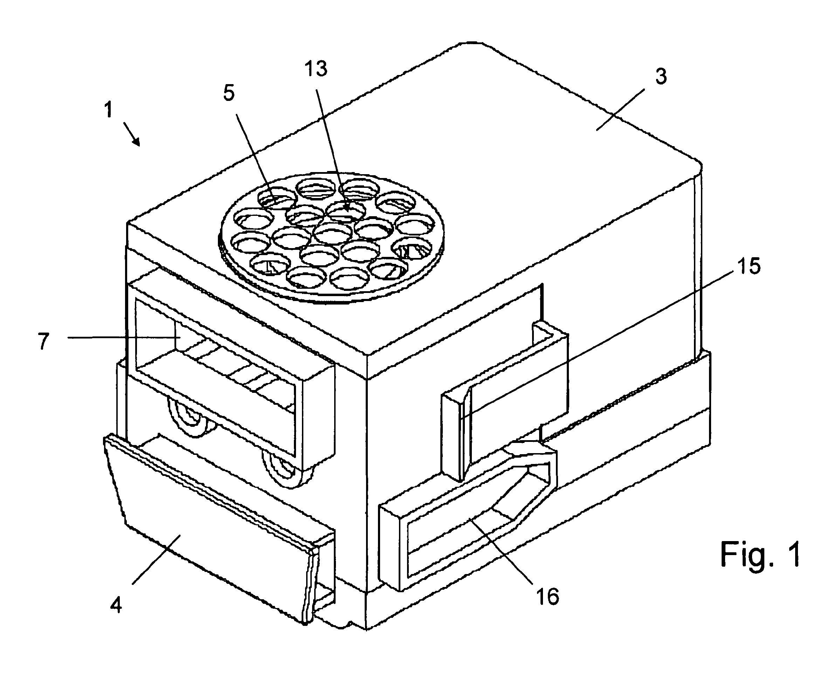

- Fig. 1: eine perspektivische Ansicht einer Beduftungsvorrichtung gemäß dem Ausführungsbeispiel,

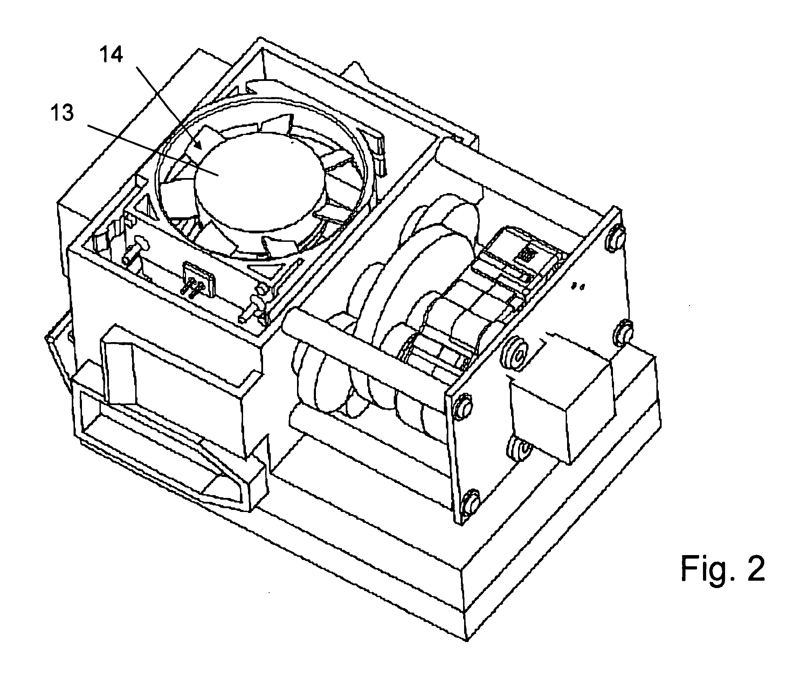

- Fig. 2: eine andere perspektivische Ansicht der Beduftungsvorrichtung von Fig. 1 ohne Abdeckung,

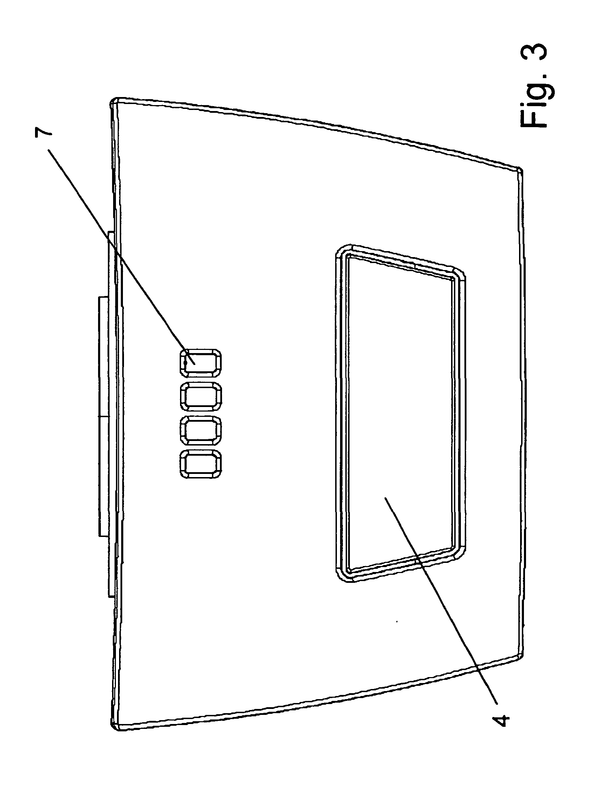

- Fig. 3: eine Frontansicht der Beduftungsvorrichtung auf die im in ein Armaturenbrett eines Fahrzeugs eingebauten Zustand sichtbare Front,

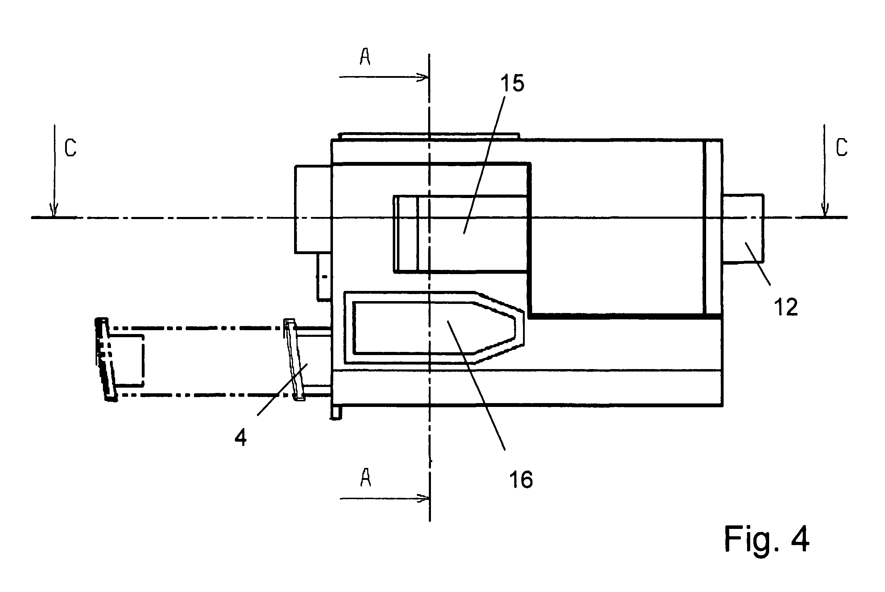

- Fig. 4: eine Seitenansicht der Beduftungsvorrichtung mit in geschlossenem und geöffnetem Zustand dargestellter Aufnahme,

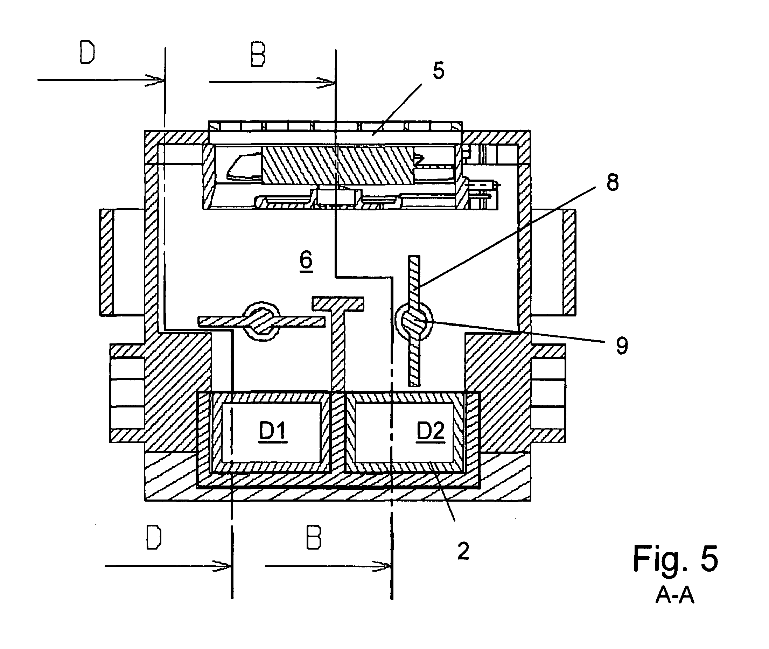

- Fig. 5: einen Schnitt entlang Linie A-A von Fig. 4,

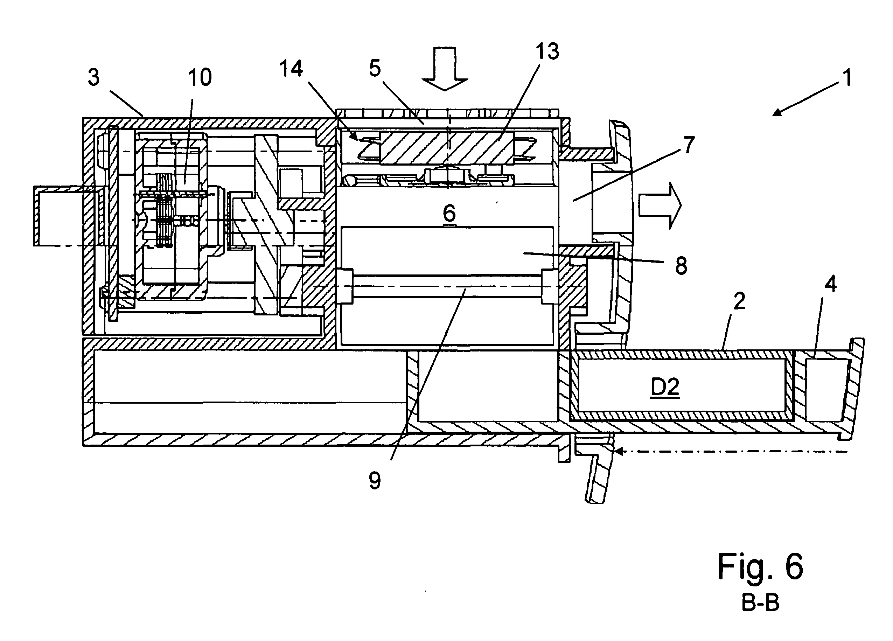

- Fig. 6: einen Schnitt entlang Linie B-B von Fig. 5 mit ausgezogener Aufnahme und Pfeilen zur Darstellung des Luftstroms,

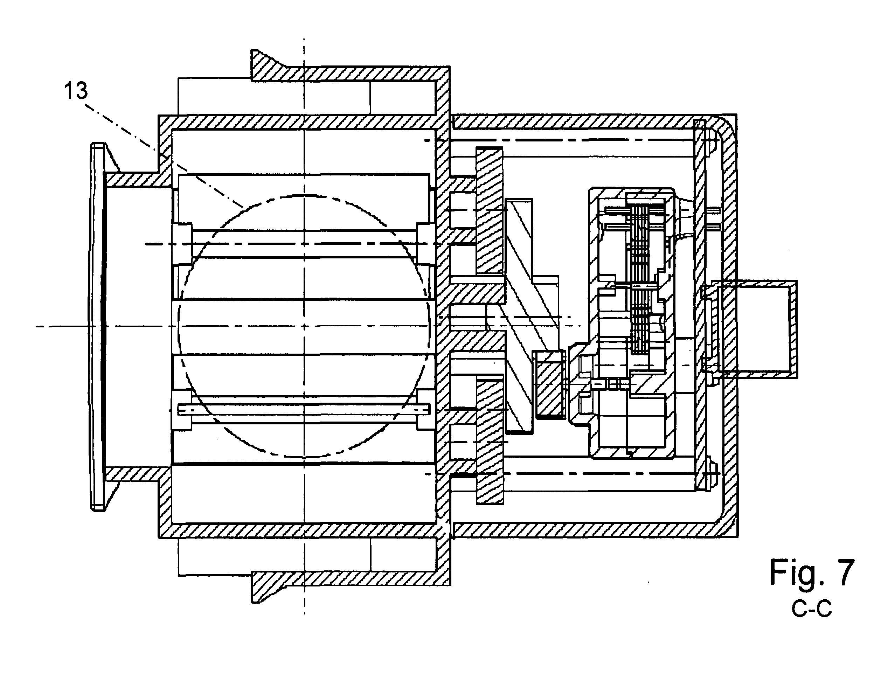

- Fig. 7: einen Schnitt entlang Linie C-C von Fig. 4,

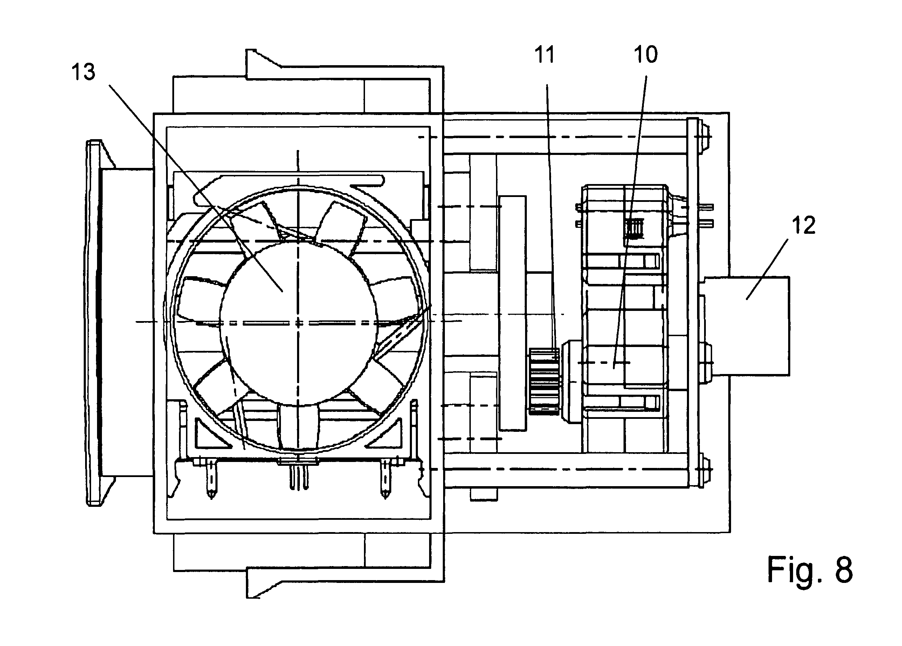

- Fig. 8: eine Fig. 7 entsprechende Draufsicht ohne Abdeckung,

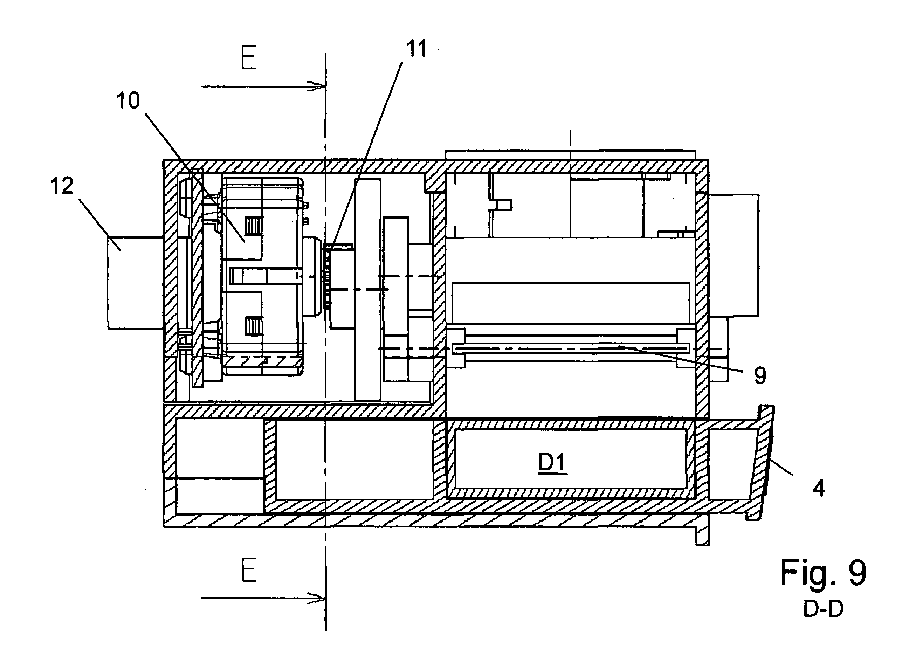

- Fig. 9: einen Schnitt entlang Linie D-D von Fig.5,

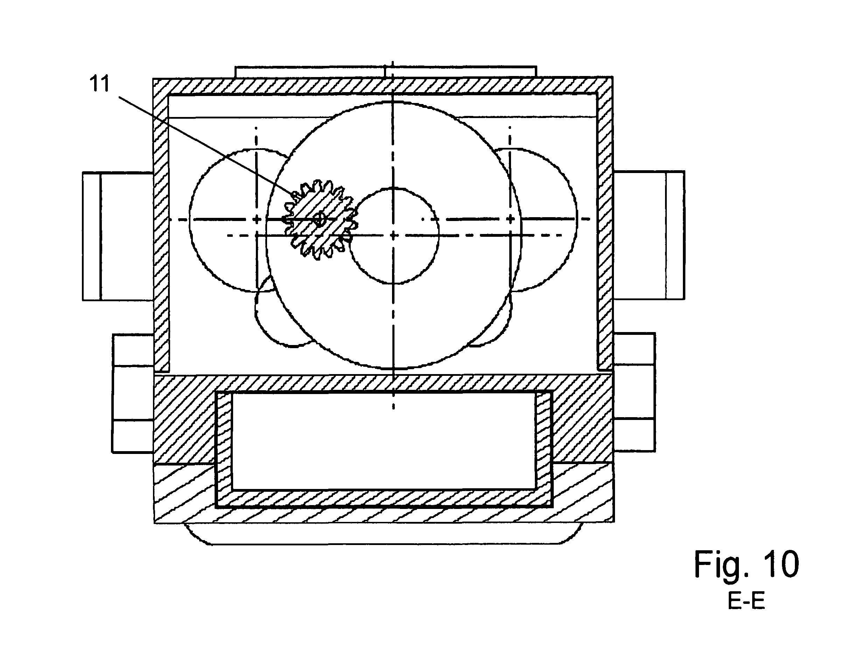

- Fig. 10: einen Schnitt entlang Linie E-E von Fig. 9,
- Fig. 11: einen Schnitt durch eine Variante in Bezug auf die Ausgestaltung der Absperrvorrichtung mit geöffneter Absperrvorrichtung,
- Fig. 12: eine Fig. 11 entsprechende Darstellung mit geschlossener Absperrvorrichtung,
- Fig.13: eine Schnittdarstellung eines zweiten Ausführunsgbeispiels einer Beduftungsvorrichtung in einer Darstellung analog Fig.6 mit eingefahrener Aufnahme,
- Fig.14: eine Schnittdarstellung des zweiten Ausführunsgbeispiels nach Fig.13 mit ausgefahrener Aufnahme,
- Fig.15: eine Schnittdarstellung des zweiten Ausführunsgbeispiels nach Fig.13 mit Dichtlippen am Gehäuse,
- Fig. 16: eine Ansicht in das Gehäuse der Beduftungsvorrichtung gemäß zweitem Ausführungsbeispiel von unten, wobei das Gehäuse aufgeschnitten und ohne Aufnahme dargestellt ist,
- Fig. 17: eine perspektivische Ansicht einer Patrone nach einem ersten Beispiel,
- Fig. 18: eine perspektivische Ansicht einer Patrone nach dem zweiten Beispiel.
- Fig. 19: ein Schnittbild durch eine Patrone nach dem zweiten Beispiel mit Deckel,
- Fig. 20a: eine perspektivische Ansicht einer Patrone nach dem zweiten Beispiel mit Deckel,
- Fig. 20b: eine perspektivische Ansicht des Deckels für die Patrone von oben nach dem zweiten Biespiel,
- Fig. 20c: eine perspektivische Ansicht des Deckels für die Patrone von unten nach dem zweiten Beispiel,
- Fig. 21: eine perspektivische Ansicht einer Patrone nach einem dritten Beispiel,
- Fig. 22: eine Draufsicht auf die Patrone nach dem dritten Beispiel,
- Fig. 23a: eine Ansicht der Patrone gemäß der Blickrichtung A in Fig. 22,
- Fig. 23b: ein Schnittbild gemäß F-F nach Fig. 23a,
- Fig. 24a: eine Seitenansicht der Patrone nach dem dritten Beispiel mit Deckel,
- Fig. 24b: eine Vorderansicht der Patrone nach dem dritten Beispiel mit Deckel,
- Fig. 24c: eine Schnittdarstellung gemäß H-H in Fig. 24a,
- Fig. 24d: eine Schnittdarstellung gemäß G-G in Fig. 24b und
- Fig. 24e: ein schematisches Schnittbild gemäß I-I in Fig. 24a.

Eine Beduftungsvorrichtung 1 zum Beduften eines Fahrzeuginnenraums mit einem Duftstoff, wobei vorliegend zwei in jeweils einer Patrone 2 enthaltene Duftstoffe D1 oder D2 zur Auswahl stehen, weist ein etwa quaderförmiges Gehäuse 3, das im Wesentlichen durch ein mehrteiliges Kunststoffspritzgussteil gebildet ist, und eine in das Gehäuse 3 einschiebbare, schubladenförmig ausgebildete Aufnahme 4 für die beiden Patronen 2 auf. Das Gehäuse 3 weist ferner eine von einer Lochplatte verschlossene Eintrittsöffnung 5, einen im Inneren des Gehäuses 3 angeordneten Mischraum 6 und eine Austrittsöffnung 7 auf. Die im eingebauten Zustand sichtbare Front ist von einer Blende verkleidet, welche an die Innenausstattung des Fahrzeuginnenraums angepasst ist, und in welcher die Austrittsöffnung 7 in Gestalt von vier nebeneinander angeordneten Öffnungen fortgeführt ist. Ebenfalls ist die Frontseite der Aufnahme 4 an die Innenausstattung des Fahrzeuginnenraums entsprechend angepasst.

Die Beduftungsvorrichtung 1 ist vorliegend in direkter Nachbarschaft eines Luftausströmers (nicht dargestellt) im Armaturenbrett eines Fahrzeugs angeordnet. Hierbei kann Luft vom zum Luftausströmer führenden Luftkanal (nicht dargestellt) durch ein kurzes Kanalstück oder, wie vorliegend vorgesehen, direkt durch eine Öffnung in der Wand des Luftkanals über die von einer Lochplatte verschlossene Eintrittsöffnung 5 in das Gehäuse 3 der Beduftungsvorrichtung 1 gelangen, wo sie sich in dem im Gehäuse 3 angeordneten Mischraum 6 mit dem entsprechenden Duftstoff D1 oder D2 vermischt und mit demselben über die kleinen Austrittsöffnungen 7 in den Fahrzeuginnenraum gelangen. Eine Verbindung mittels eines Luftkanals kann auch entfallen, so dass Umluft beziehungsweise Umgebungsluft direkt durch die Eintrittsöffnung 5 in das Gehäuse 3 der Beduftungsvorrichtung 1 gelangt. Durch die durch den Luftausströmer ausströmende Luft wird die hierzu benachbart ausströmende, mit dem Duftstoff D1 und/oder D2 versetzte Luft schnell im Fahrzeuginnenraum verteilt. Die in der Aufnahme 4 angeordneten Patronen 2 sind durch Klappen 8 vom Mischraum 6 abtrennbar, welche als Absperrvorrichtung dienen. Die Klappen 8 sind um Schwenkachsen 9 verschwenkbar, welche parallel zur Einschubrichtung der Aufnahme 4 verlaufen. Die Klappen 8 sind vorliegend mittels eines Schrittmotors 10, dessen Stellbewegungen über ein Getriebe 11 übersetzt werden, betätigbar. Der Schrittmotor 10 ist über einen elektrischen Steckkontakt 12, welcher am Gehäuse 3 ausgebildet ist, mit dem Bordnetz und der Regelung verbindbar. Die Betätigung der Klappen 8 erfolgt vorliegend in Abhängigkeit der vorgesehenen Regelung im Wesentlichen unabhängig voneinander, d.h. es können beide Klappen geschlossen oder geöffnet sein, oder es kann nur eine der Klappen geöffnet und die andere Klappe geschlossen sein. Gemäß dem vorliegenden Ausführungsbeispiel sind keine Zwischenstellungen der Klappen vorgesehen, d.h. die Klappen 8 sind entweder vollständig geöffnet oder vollständig geschlossen.

Gemäß einer Variante des Ausführungsbeispiels sind an Stelle von zwei Patronen 2 in der Aufnahme 4 und zwei Klappen 8 im Gehäuse 3 nur eine einzige Patrone und eine einzige Klappe vorgesehen. Gemäß einer weiteren Variante des Ausführungsbeispiels sind drei Patronen in der Aufnahme und drei Klappen im Gehäuse vorgesehen, so dass drei Duftstoffe und - bei stufenlos verstellbaren Klappen - entsprechend viele Mischungen/Mischungsverhältnisse zur Verfügung stehen.

Um die Funktion des Mischraums 6 zu verbessern und die Bewegungsenergie der einströmenden, vom Luftkanal kommenden Luft zu nutzen, ist im Bereich zwischen der von einer Lochplatte verschlossenen Eintrittsöffnung 5 und dem Mischraum 6 ein Laufrad 13 (vorliegend ein elektrisch angetriebener Axiallüfter) angeordnet, welches durch seine Drehbewegung die einströmende Luft mit einem zusätzlichen Drall versetzt, welcher ein Vermischen des Duftstoffs D1 oder D2 mit der eingeströmten Luft verbessert, d.h. das Laufrad 13 dient als Drallerzeugungsvorrichtung 14.

Das Einströmen in den Mischraum kann jedoch auch in einer geradlinig gerichteten Strömung erfolgen, wobei zur Beruhigung Leitprofile vorgesehen sind.

Die Beduftung kann kontinuierlich erfolgen, d.h. die Klappe 8 zum entsprechenden Duftstoff D1 oder D2 ist über den gesamten Zeitraum der Beduftung geöffnet. Alternativ, insbesondere um einen Gewöhnungseffekt zu vermeiden, kann die Klappe getaktet geöffnet werden. Die Stellbewegungen der Klappe 8 erfolgen, wie vorstehend erwähnt, mittels des Stellmotors 10, dessen Antriebsbewegung durch das Getriebe 11 bedarfsgerecht umgewandelt und an die Welle(n) der Klappen 8 übertragen wird.

Gemäß einer alternativen Ausführungsform sind an Stelle des frei drehbaren Laufrades entsprechend angeordnete Leitschaufeln vorgesehen, welche die Funktion als Drallerzeugungsvorrichtung 14 übernehmen.

Das Gehäuse 3 ist an seinen seitlichen Außenseiten mit je einem Federarm 15 und einer Führung 16 ausgebildet, welche dem einfachen Einbau und der Fixierung im Armaturenbrett dienen. Die Außenabmessungen lassen sich so Standardisieren, d.h. unterschiedliche Beduftungsvorrichtungen können in die entsprechenden Öffnungen im Armaturenbrett eingesetzt werden. Die Front lässt sich durch einfaches Auswechseln der Blende an das Armaturenbrett anpassen.

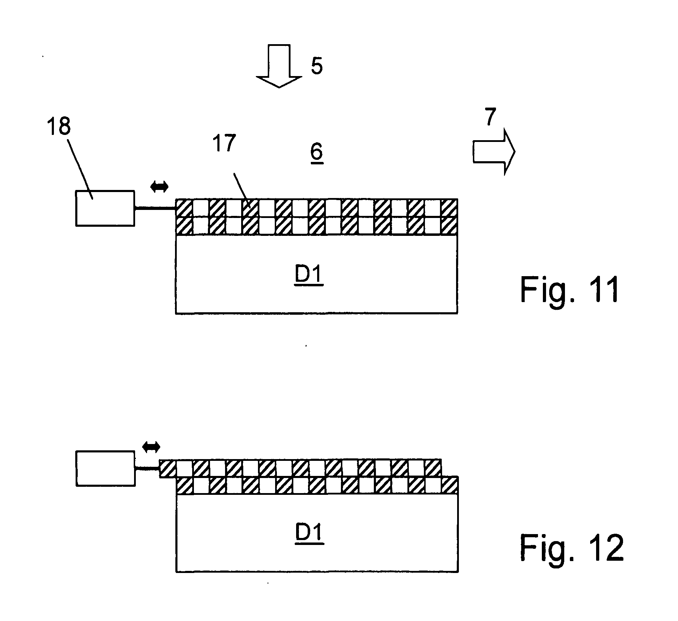

Die Absperrvorrichtung, welche gemäß dem vorstehend beschriebenen Ausführungsbeispiel durch Klappen 8 gebildet ist, welche durch den Schrittmotor betätigbar sind, kann alternativ, wie in Figuren 11 und 12 dargestellt, durch zwei relativ zueinander verschiebbare, geschlitzte Bleche 17 gebildet sein, wobei die Schlitzbreiten maximal so groß wie die Stegbreiten sind, so dass ein vollständiges gegenseitiges Verschließen der Öffnungen möglich ist. Die Betätigung kann mittels eines Hubmagneten 18 erfolgen, an welchen eine Spannung anlegbar ist, so dass dieser eines der beiden Bleche 17 in Längsrichtung relativ zum anderen bewegt, das fest oberhalb der Aufnahme, in welcher der Duftstoff D1 angeordnet ist, positioniert ist. Die Ausgestaltung der Beduftungsvorrichtung entspricht ansonsten der zuvor beschriebenen Beduftungsvorrichtung 1.

An Stelle eines metallischen Blechs kann auch eine entsprechend ausgebildete Kunststoffplatte oder eine Platte aus einem anderen geeigneten Material vorgesehen sein.

Alternativ zu Schlitzen können auch Bohrungen o.ä. im Blech oder der Platte vorgesehen sein, welche im geöffneten Zustand miteinander fluchten, im geschlossenen Zustand aber jeweils von einer geschlossenen Fläche des anderen Elements verdeckt werden.

Gemäß einer nicht in der Zeichnung dargestellten Variante in Bezug auf die Absperrvorrichtung ist als Klappe eine Jalousieklappe vorgesehen, welche Öffnungen aufweist, welche im geöffneten Zustand mit anderen Öffnungen fluchten, im geschlossenen Zustand jedoch jeweils durch eine gegenüberliegende Fläche verdeckt sind. Ebenfalls kann an Stelle von einer einzigen Klappe je Duftstoff D1 und/oder D2 eine Mehrzahl von kleineren Klappen vorgesehen sein. Diese ermöglichen eine feinere Dosierung der Duftstofffreisetzung indem in Zwischenzuständen nur ein Teil der Klappen geöffnet werden.

Um die Patronen 2 einfach wechseln zu können, ist die Aufnahme 4 am in Einschubrichtung vorderen Ende einer Feder (nicht dargestellt) angeordnet, welche - bei Lösen einer Verriegelung in Folge eines Drückens auf die Frontfläche der Aufnahme 4 - die Aufnahme 4 automatisch ausfährt und die Patronen 2 derart freigibt. Wird die Aufnahme 4 wieder eingeschoben, so verrastet die Aufnahme 4 automatisch im Gehäuse 3. Andere konstruktive Lösungen sind ebenfalls möglich.

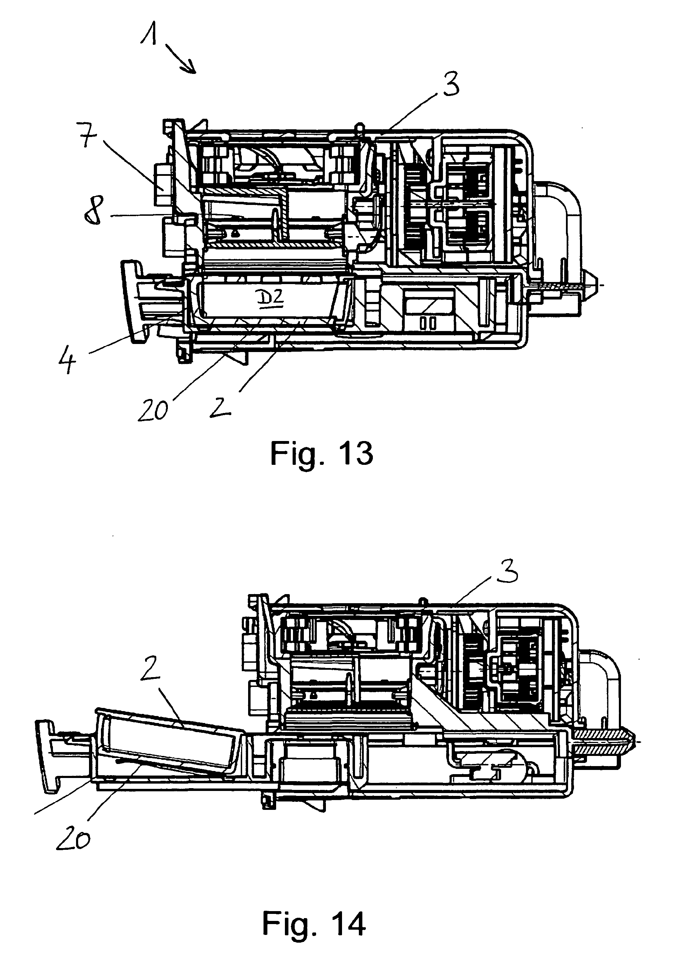

In den Figuren 13 und 14 ist ein zweites Ausführungsbeispiel einer Beduftungsvorrichtung dargestellt, das dem ersten Ausführungsbeispiel im funktionalen Aufbau im Wesentlichen entspricht. Alle funktionell gleichwirkenden Komponenten mit gleichem oder ähnlichem Aufbau sind mit denselben Bezugszeichen wie in der Beschreibung des ersten Ausführungsbeispiels versehen. In Fig. 13 ist die schubladenförmig gebildete Aufnahme 4 für die Patrone 2, welche den Duftstoff enthält, in einer in das Gehäuse 3 eingeschobenen Position gezeigt. Um die Patrone 2 zu wechseln wird die Aufnahme 4 aus dem Gehäuse heraus bewegt. Dies erfolgt vorteilhafterweise, wie im ersten Ausführungsbeispiel beschrieben, durch Lösen einer Verriegelung mittels Drückens auf die Frontfläche der Aufnahme 4, wobei die Aufnahme 4 automatisch über eine auf die vordere Seitenwand der Aufnahme drückende Feder ausfährt und die Patronen 2 freigibt. Wie insbesondere in Fig.14 zu erkennen ist an der Bodenfläche der Aufnahme 4 eine Auswurffeder 20 angeordnet, die die Patrone 2 im ausgefahrenen Zustand schräg nach oben anhebt und so eine leichtere Entnahme der Patrone 2 ermöglicht. Alternativ kann eine entsprechende Feder 20 auch an der Patrone 2 selbst vorgesehen sein.

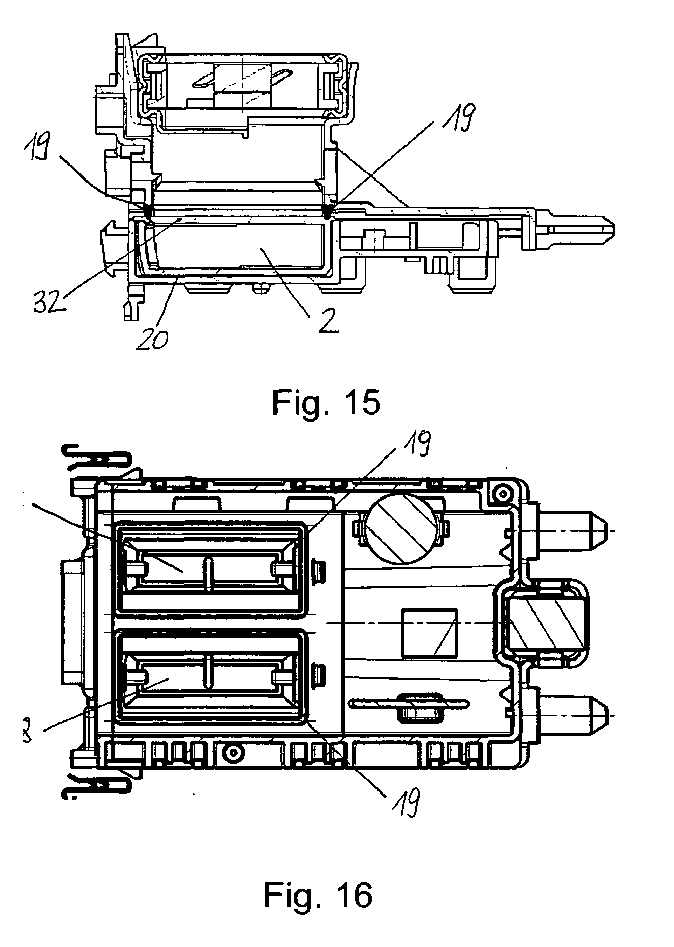

Fig. 15 zeigt eine Schnittdarstellung des zweiten Ausführungsbeispiels analog zur Darstellung in Fig. 13, wobei Aktuatorik und Getriebeeinheit im rechten oberen Teil der Beduftungsvorrichtung weggelassen wurde. Am Bereich des Gehäuses 3, in welchen die Patrone 2 mit dem Aufnahmeschacht 4 eingeschoben wird, ist eine Dichtlippe 19 ausgebildet. Die Auswurffeder 20 drückt das Oberteil bzw. das Abdeckteil 32 der Patrone 2 gegen die Dichtlippe 19 an, welche so ausgebildet ist, dass sie bei eingeschobener Position der Aufnahme 4 umlaufend am Abdeckteil 32 der Patrone anliegt und die Öffnungen in diesem einschließt, sodass eine Abdichtung zwischen der Patrone 2 und dem Gehäuse 3 gewährleistet ist. In Fig. 16 sind die umlaufend ausgebildeten Dichtlippen 19 in einer aufgeschnittenen Ansicht von unten in das Gehäuse 3 deutlich zu erkennen. Bedingt durch die rechteckige Form der Patronen, ist auch die Dichtlippe 19 in der Form eines Rechtecks verlaufend ausgebildet, wobei die Ecken abgerundet sind.

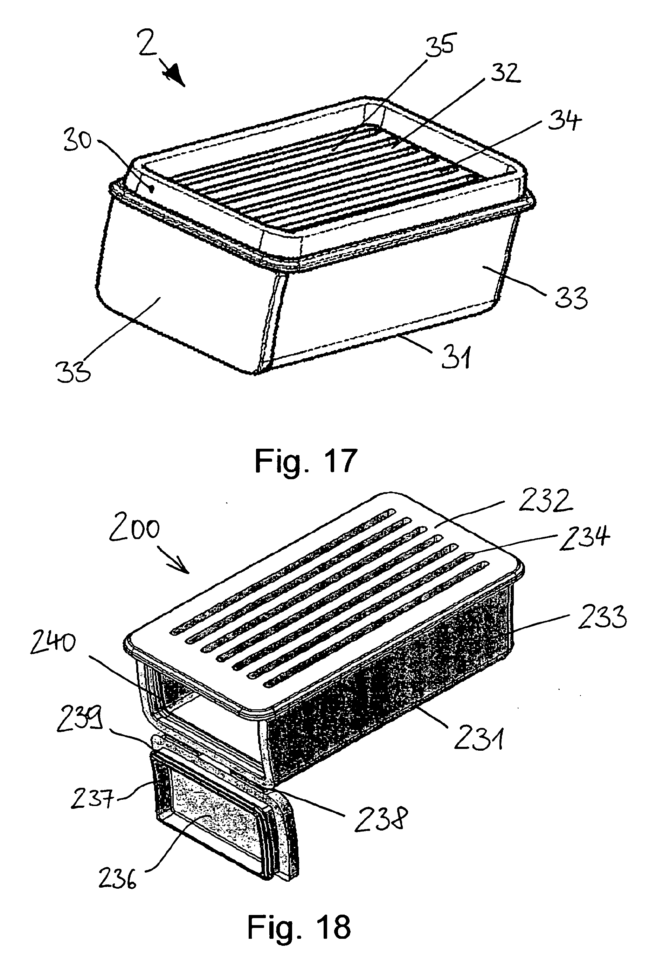

Ein erstes Beispiel einer Patrone 2 oder Kartusche, die einen Duftstoff enthält und für den Gebrauch in einer Beduftungsvorrichtung vorgesehen ist, ist in Fig. 17 dargestellt. Die Patrone weist ein Bodenteil 31, ein Abdeckteil 32 und das Boden- und Abdeckteil verbindende Seitenwände 33 auf, die einen Raum umschließen in dem der Duftstoff oder der Duftstoffträger mit einem Duftstoff aufgenommen ist. Die Patrone 2 ist im Wesentlichen quaderförmig ausgebildet. Das Abdeckteil 32 weist schlitzförmige Öffnungen 34 auf, wobei die Stege 35 zwischen den Öffnungen, sowie die Öffnungen selbst eine Breite von 1 mm aus Festigkeits- und Herstellgründen nicht unterschreiten. Alle anderen Wände wie Bodenteil 31 und Seitenwände 33 sind geschlossen, d.h. ohne Öffnungen ausgebildet. Am Abdeckteil 32 ist eine umlaufende Dichtlippe 30 vorgesehen, die mit einem entsprechenden Gehäusebauteil einer Beduftungsvorrichtung zusammenwirkt und einen ungewünschten Duftaustritt nach außen verhindert. Für den Fall, dass eine entsprechende Dichtlippe 19, wie im zweiten Beispiel der Beduftungsvorrrichtung am Gehäuse ausgebildet ist, kann die Dichtlippe 30 an der Patrone 2 auch entfallen.

In einem zweiten Beispiel einer Patrone 200, ist eine der Seitenwände 233 als Klappwand 236 ausgebildet. Die Klappwand 236 ist mittels eines Filmscharniers 238 einstückig und einteilig mit dem Bodenteil 231 der Patrone 200 verbunden und für den Austausch des Duftstoffs bzw. des Duftstoffträgers wegklappbar. Eine solche weggeklappte Stellung der Klappwand 236 ist in Fig. 18 dargestellt. An der Innenseite der Klappwand ist ein rechteckförmig verlaufender Steg 237 vorgesehen an dessen Endbereich sich ein Vorsprung 239 befindet, der beim Schließen der Patrone durch Hochklappen der Klappwand 236 an einer Leiste 240, die ebenfalls umlaufend im Inneren der Patrone ausgebildet ist, einrastet. Die gesamte Patrone 200, lässt sich im Spritzgußverfahren, besonders vorteilhaft einstückig und einteilig, herstellen.

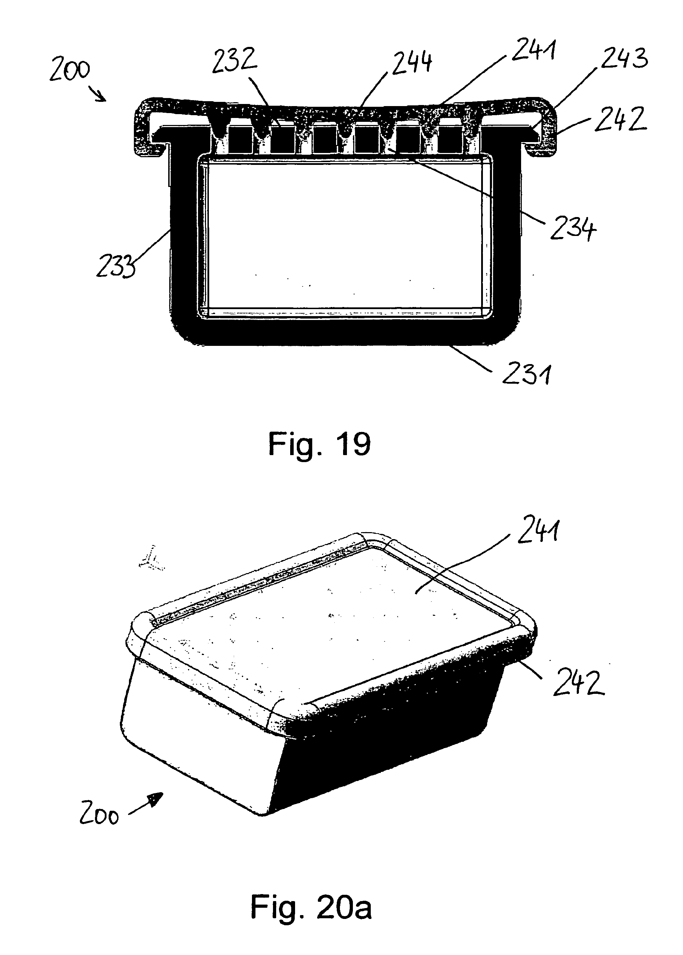

In einer Lager- oder Transportposition gemäß Fig. 19, also in einem Zustand, in welchem die Patrone nicht in eine Beduftungsvorrichtung eingesetzt ist, ist die Patrone mittels eines Deckels 241, der einen hakenförmigen Rand 242 aufweist und in entsprechende Vorsprünge 243 am Abdeckteil 232 eingreift, verschlossen. Sowohl der hakenförmige Rand 242 des Deckels 241, als auch der Vorsprung 243 am Abdeckteil 232 sind für eine gute Dichtung umlaufend ausgebildet. Um die Abdichtung weiter zu verbessern, sind an der Unterseite des Deckels 241, Rippen 244 vorgesehen, die in ihrer Form an die Öffnungen 234 des Abdeckteils 232 angepasst sind und in diese hineinragen. Die Rippen sind dabei leicht konisch ausgeführt, so dass sie dichtend in den Öffnungen 234 anliegen und durch die Verbindung des hakenförmigen Randes 242 mit den Vorsprüngen 243 an diese gepresst werden. Die Deckeloberfläche ist leicht nach innen gekrümmt ausgeführt, so dass ein Verrasten des hakenförmigen Randes 242 an dem oder den Vorsprüngen 243 am Abdeckteil unter Vorspannung möglich ist. Gemäß einer alternativen nicht in der Figur dargestellten Ausführungsform, sind die Rippen 244 nur für ausgewählte Öffnungen 234, beispielsweise nur die beiden randseitigen Öffnungen 234 vorgesehen.

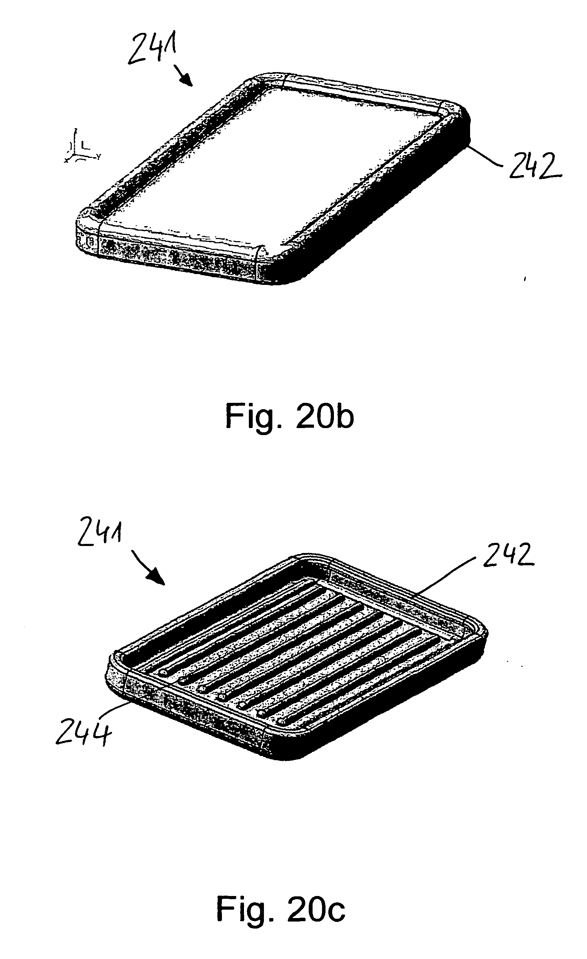

Fig. 20a zeigt die Patrone 200 gemäß dem zweiten Beispiel mit dem Deckel 241 in einer perspektivischen Darstellung. Der Deckel 241 ist in perspektivischer Draufsicht in Fig. 20b gezeigt, wobei der umlaufende Rand zu erkennen ist. Eine perspektivische Ansicht der Unterseite des Deckels 241 ist Fig. 20c zu entnehmen. Der umlaufende hakenförmige Rand 242 ist im wesentlichen rechteckförmig verlaufend ausgebildet, wobei die entsprechenden Ecken abgerundet sind. Im Innenbereich sind die Rippen 244 mit konus- oder halbkreisförmigen Profil zur Aufnahme in den Öffnungen 234 angeordnet.

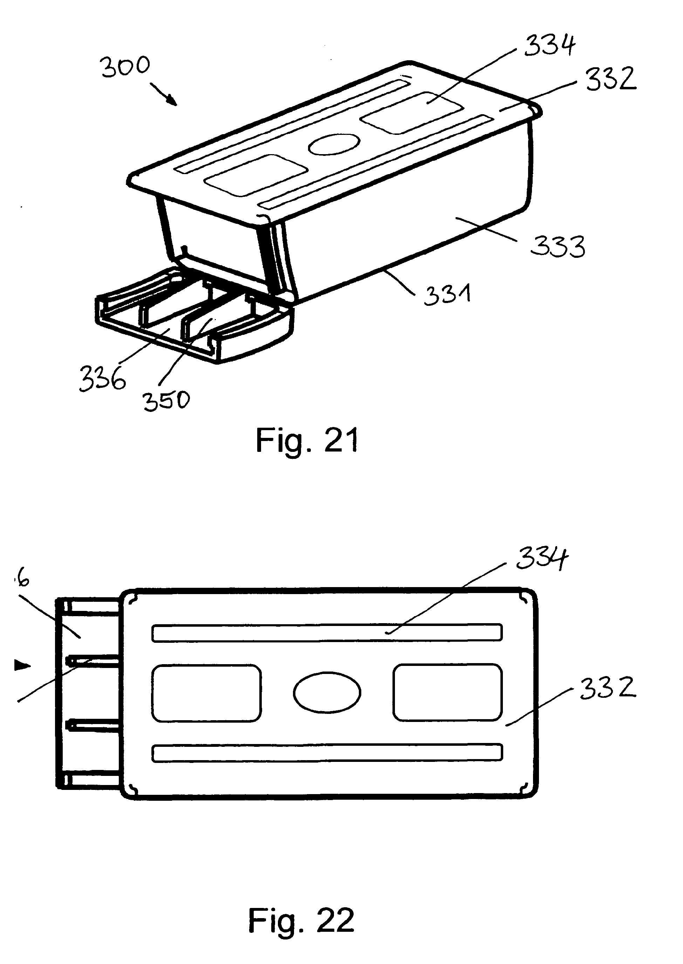

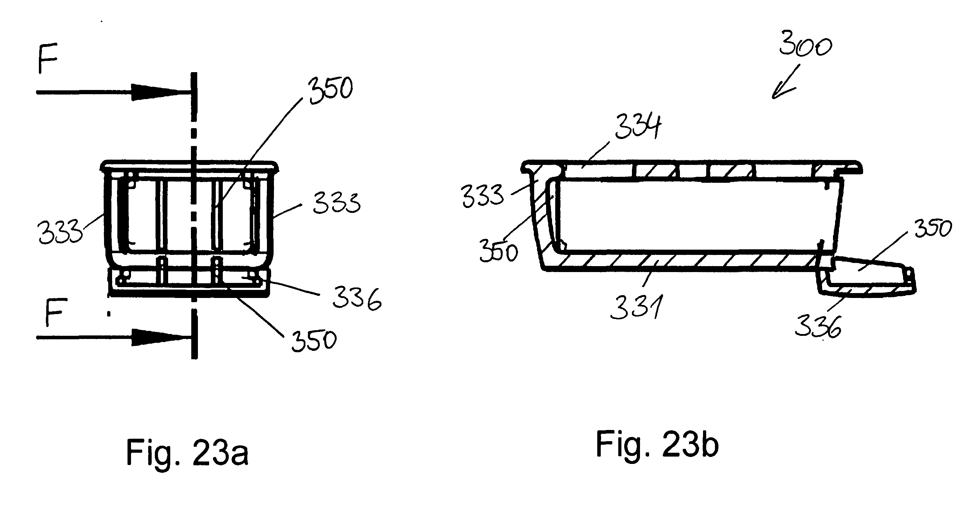

Ein drittes Beispiel einer erfindungsgemäßen Patrone 300 ist in Fig. 21 dargestellt. Im Unterschied zum zweiten Beispiel sind einige der Öffnungen 334 verschiedenartig geformt. D.h. die Öffnungen im Abdeckteil 332 weisen nicht mehr wie im ersten und zweiten Beispiel gleiche Form auf, sondern sind zumindest teilweise unterschiedlich ausgebildet. Insbesondere in der Draufsicht in Fig. 22 ist zu erkennen, dass vorliegend zwei schlitzartige Öffnungen an den Rändern und dazwischenliegend zwei rechteckförmige und eine elliptische Öffnung 234 ausgebildet sind. Die Gesamtfläche der Öffnungen 334 im Abdeckteil 332 beträgt vorliegend 50% der Fläche des Abdeckteils 332. Wie im vorhergehenden Bespiel ist eine Klappwand 336 mit dem entsprechenden Verschlussmechanismus vorgesehen. Zusätzlich sind an der Innenwand der Klappwand 336 zwei Stützrippen 350 vorgesehen, welche der Klemmung des in der Patrone aufgenommen Duftstoffträgers, wie beispielsweise eines duftstoffangereicherten Vlieses oder eines Filzkörpers, dienen. Selbstverständlich können statt zweier Stützrippen 350 auch mehr oder weniger vorgesehen sein. Wie den Darstellungen in Fig.23a und Fig. 23b zu entnehmen, sind auch an der, der Klappwand 336 gegenüberliegenden Seitenwand 333 entsprechende Stützrippen 350 angeordnet.

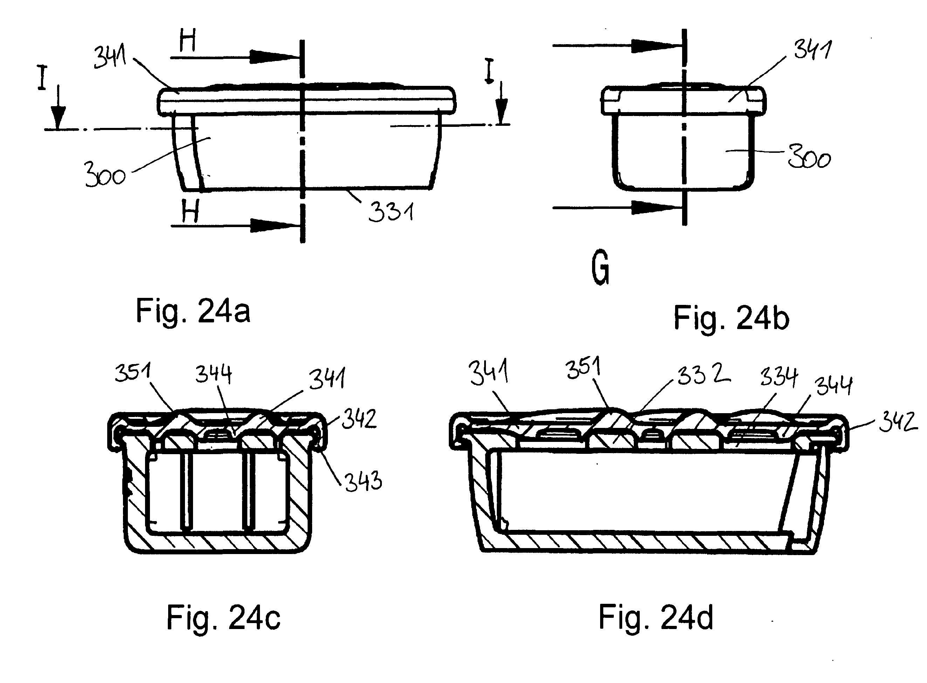

Mittels der Schnittdarstellungen in den Fig. 24c und 24d ist gezeigt, wie der Eingriff der Rippen 344 des Deckels 341 in die Öffnungen 334 des Abdeckteils 332 erfolgt. Die entsprechenden Rippen 344 sind wie im zweiten Beispel an die Form der Öffnungen 334 angepasst, wobei die Kontur bzw. der Umriss der Rippen 344 im Wesentlichen der Kontur bzw. der Umriss der Öffnungen 334 entspricht. Zur Versteifung sind auf der Oberseite des Deckels 341 entsprechende Erhöhungen oder Ausbuchtungen 351 vorgesehen. An den Seitenrändern des Deckels 341 umgreifen hakenförmige Ränder 342 entsprechende Vorsprünge 343 des Abdeckteils 332, sodass es zu einem luftdichten Verschluss kommt. Wie der Fig. 24a zu entnehmen, verlaufen die Seitenwände 333 an der Vorder- und Rückseite der Patrone nicht senkrecht zum Bodenteil, sondern weisen eine konvexe Krümmung vom Abdeckteil 332 in Richtung Bodenteil 331 auf. Dies erleichtert das Einführen und die Entnahme der Patrone 300 in die entsprechende Aufnahme der Beduftungsvorrichtung.

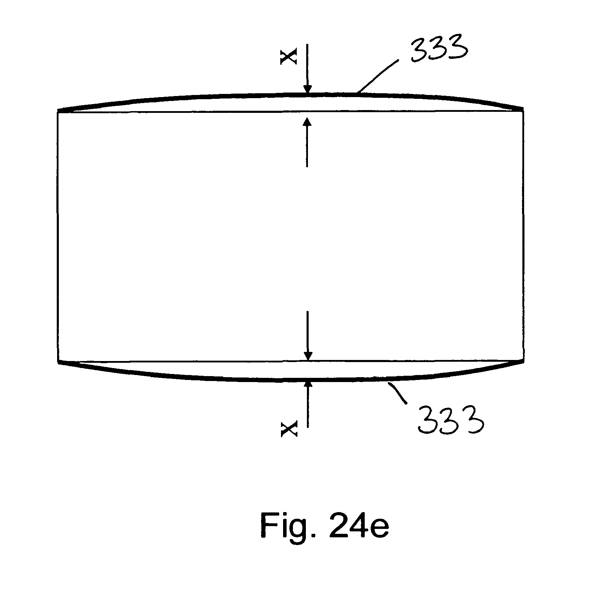

Fig. 24e verdeutlicht, dass die zwei gegenüberliegenden, vorliegend längeren Seitenwände, der im wesentlichen rechteckförmigen Patrone nicht genau parallel verlaufend ausgebildet sind. Zum Vergleich ist in Fig. 24e ein Rechteck eingetragen, dass die Abweichung der Seitenwände von dieser Form illustriert und insbesondere die entsprechenden Ausbauchung der Seitenwände 333 darstellt. Das Maß x beträgt an beiden Seiten 0.5 mm und gibt die Abweichung von der Parallelität an. Über die Länge der beiden gegenüberliegenden Seitenwände 333, betrachtet, bedeutet dies, das ein entsprechender Normalabstand der Seitenwände 333 jeweils von den Vorder- und Hinterseiten der Patrone aus zur Mitte hin zunimmt und die Ausbauchung in der Mitte somit den Maximalwert annimmt. Da vorliegend die Seitenwände nicht genau parallel sind, ist unter dem Normalabstand der jeweilige Abstand zwischen zwei Punkten der gegenüberliegenden, ausgebauchten Seitenwände, gemessen parallel zur vorderen oder rückwertigen Seitenwand 333 zu verstehen. Auch diese Ausführung, kann selbstverständlich in allen Ausführungsbeispielen vorgesehen sein. Der Einsatz in den Aufnahmeschacht 4 einer Beduftungsvorrichtung 1 wird dadurch erleichtert und insbesondere eine klemmende Fixierung ermöglicht. Alternativ oder zusätzlich können an den Seitenflächen auch mehrere Noppen vorgesehen sein um einen Klemmsitz in der Aufnahme zu verbessern.

### Bezugszeichenliste

- 1: Beduftungsvorrichtung
- 2, 200, 300: Patrone
- 3: Gehäuse
- 4: Aufnahme
- 5: Eintrittsöffnung
- 6: Mischraum
- 7: Austrittsöffnung
- 8: Klappe
- 9: Schwenkachse
- 10: Schrittmotor
- 11: Getriebe
- 12: Steckkontakt
- 13: Laufrad
- 14: Drallerzeugungsvorrichtung
- 15: Federarm
- 16: Führung
- 17: Blech
- 18: Hubmagnet
- 19: Dichtlippe am Gehäuse
- 20: Auswurffeder
- 30: Dichtlippe auf Patrone
- 31, 231, 331: Bodenteil
- 32, 232, 332: Abdeckteil
- 33, 233, 333: Seitenwand
- 34, 234, 334: Öffnung in Abdeckteil
- 35: Steg in Abdeckteil
- 37: Steg
- 38: Filmscharnier
- 39: Vorsprung
- 41: Deckel
- 43: Vorsprung
- 236, 336: Klappwand
- 237: Steg an Klappwand
- 238: Filmscharnier
- 239: Vorsprung
- 240: Leiste
- 241, 341: Deckel
- 242, 342: hakenförmiger Rand
- 244, 344: Rippe an Deckel
- 350: Stützrippe
- 351: Erhöhungen

- D1, D2: Duftstoff

## Patentansprüche

1. Beduftungsvorrichtung, insbesondere für ein Fahrzeug, mit einem Gehäuse (3), zumindest einer in einer im Gehäuse (3) vorgesehenen Aufnahme (4) angeordneten Patrone (2, 200, 300), in welcher ein Duftstoff (D1 oder D2) enthalten ist, einer Eintrittsöffnung (5), über welche Luft in das Gehäuse (3) der Beduftungsvorrichtung (1) gelangen kann, einem Mischraum (6), in welchem der Duftstoff (D1 und/oder D2) mit der Luft vermischbar ist, einer Austrittsöffnung (7), über welche die mit dem Duftstoff (D1 und/oder D2) vermischte Luft nach außen gelangen kann, und mindestens einer betätigbaren Absperrvorrichtung, welche den Duftstoff (D1 und/oder D2) an einem Austreten aus der Aufnahme (4) in den Mischraum (6) in mindestens einer Stellung hindert, wobei im Bereich der Eintrittsöffnung (5) und/oder zwischen der Eintrittsöffnung (5) und dem Mischraum (6) eine Drallerzeugungsvorrichtung (14) vorgesehen ist, wobei die Absperrvorrichtung zwischen der Aufnahme (4) und dem Mischraum (6) angeordnet ist und die Absperrvorrichtung durch eine Klappe (8) gebildet ist, **dadurch gekennzeichnet, dass** die Aufnahme (4) für die Patrone oder Patronen (2, 200, 300) schubladenförmig ausgebildet ist, am Gehäuse (3) eine oder mehrere Dichtlippen (19) angeordnet sind, die an einer Fläche der Patrone (32, 232, 332) zur Abdichtung anliegen und die Aufnahme (4) für die Patrone oder Patronen (2, 200, 300) auf der der Drallerzeugungsvorrichtung (14) gegenüberliegenden Seite des Mischraums (6) angeordnet ist, wobei die Schwenkachse (9) der Klappe (8) parallel zur Einschubrichtung der Aufnahme (4) verläuft.

2. Beduftungsvorrichtung nach Anspruch 1, **dadurch gekennzeichnet, dass** die Drallerzeugungsvorrichtung (14) durch ein drehbares Laufrad (13) und/oder starre Leitschaufeln gebildet ist.

3. Beduftungsvorrichtung nach Anspruch 1 oder 2, **dadurch gekennzeichnet, dass** die Aufnahme (4) federvorgespannt ist.

4. Beduftungsvorrichtung nach einem der Ansprüche 1 bis 3, **dadurch gekennzeichnet, dass** die Absperrvorrichtung durch zwei relativ zueinander verschiebbare, mit Öffnungen versehene Elemente oder durch eine Jalousieklappe gebildet ist.

5. Beduftungsvorrichtung nach einem der vorhergehenden Ansprüche mit der Patrone (2, 200, 300) zur Freigabe eines Duftstoffes aufweisend ein Bodenteil (31, 231, 331), ein Abdeckteil (32, 232, 332) und das Boden- und Abdeckteil verbindende Seitenwände (33, 233, 333), die einen Raum umschließen in dem ein Duftstoff oder ein Duftstoffträger mit einem Duftstoff aufgenommen ist, wobei in dem Abdeckteil (32, 232, 332) eine oder mehrere Öffnungen (34, 234, 334) vorgesehen sind, durch die der Duftstoff austreten kann.

6. Beduftungsvorrichtung nach Anspruch 5, **dadurch gekennzeichnet, dass** die Gesamtfläche der Öffnungen (34, 234, 334) im Abdeckteil (32, 232, 332) kleiner ist als 80%.

7. Beduftungsvorrichtung nach Anspruch 5 oder 6, **dadurch gekennzeichnet, dass** am Abdeckteil eine Dichtlippe (30) angeordnet ist, die umlaufend am Randbereich verläuft.

8. Beduftungsvorrichtung nach einem der Ansprüche 5 bis 7, **dadurch gekennzeichnet, dass** zwei gegenüberliegende Seitenwände (33, 233, 333) nicht genau parallel verlaufen.

9. Beduftungsvorrichtung nach einem der Ansprüche 5 bis 8, **dadurch gekennzeichnet, dass** zumindest eine Seitenwand (36, 236, 336) abnehmbar oder am Abdeck- oder Bodenteil schwenkbar ausgebildet ist.

10. Beduftungsvorrichtung nach einem der Ansprüche 5 bis 9, **dadurch gekennzeichnet, dass** die Patrone (2, 200, 300) in einer Lager- oder Transportposition mittels eines Deckels (241, 341), der einen hakenförmigen Rand (242, 342) aufweist und entsprechende Vorsprünge (243, 343) am Abdeckteil (232, 332) umgreift, verschlossen ist.

## Claims

1. An hair treatment device, in particular for a motor vehicle, comprising a housing (3), at least one cartridge (2, 200, 300), which is disposed in a receptacle (4) provided in the housing (3) and in which a fragrance (D1 or D2) is contained, an inlet opening (5), via which air can center the housing (3) of the air treatment device (1), a mixing chamber (6), in which the fragrance (D1 and/or D2) can be mixed with the air, an outlet opening (7), via which the air mixed with the fragrance (D1 and/or D2) can reach the outside, and at least one actuatable blocking device, which, in at least one Position, blocks the fragrance (D1 and/or D2) from emerging from the receptacle (4) and entering the mixing chamber (6), wherein a swirl-generating device (14) is provide in the region of the inlet opening (5) and/or between the inlet opening (5) and the mixing chamber (6), wherein the blocking device is disposed between the receptacle (4) and the mixing chamber (6) and the blocking device is formed by a flap (8),
**characterized in that** the receptacle (4) for the cartridge or cartridges (2, 200, 300) is designed in the shape of a drawer, once or more sealing lips (19) are disposed on the housing (3) and bear against a surface of the cartridge (32, 232, 332) for sealing, and the receptacle (4) for the cartridge or cartridges (2, 200, 300) is disposed on the side of the mixing chamber (6) apposite the swirl-generating device (14), wherein the swivelling axis (9) of the flap (8) extends parallel to the insertion direction of the receptacle (4).

2. The air treatment device according to claim 1, **characterized in that** the swirl-generating device (14) is formed by a rotatable blower wheel (13) and/or rigid guide blades.

3. The device according to claim 1 or 2, **characterized in that** the receptacle (4) is spring-loaded.

4. The air treatment device according to one of the claims 1 to 3, **characterized in that** the blocking device is formed by two elements, which are provided with openings and are displaceable relative to one another, or by a multiflap shutter.

5. The air treatment device according to one of the preceding claims, comprising the cartridge (2, 200, 300) for releasing a fragrance/having a base part (31, 231, 331), a cover part (32, 232, 332) and side walls (33, 233, 333), which connect the base part and the cover part and enclose a space in which a fragrance or a fragrance carrier having a fragrance is accommodated, wherein one or more openings (34, 234, 334) through which the fragrance can emerge are provided in the cover part (32, 232, 332).

6. The air treatment device according to claim 5, **characterized in that** the total surface area of the openings (34, 234, 334) in the cover part (32, 232, 332) is less than 80%.

7. The air treatment device according to claim 5 or 6, **characterized in that** a sealing lip (30), which extends circumferentially at the edge region, is disposed on the cover part.

8. The air treatment device according to one of the claims 5 to 7, **characterized in that** two opposing side walls (33, 233, 333) do not extend exactly parallel.

9. The air treatment device according to one of the claims 5 to 8, **characterized in that** at least one side wall (36, 236, 336) is removable or is swivellable at the cover part or the base part.

10. The air treatment device according to one of the claims 5 to 9, **characterized in that** the cartridge (2, 200, 300) is closed in a storage or transport position by way of a cover (241, 341), which has a hook-shaped edge (242, 342) and engages around corresponding projections (243, 343) on the cover part (232, 332).

## Revendications

1. Dispositif servant à parfumer, en particulier pour un véhicule, comprenant un boîtier (3), au moins une cartouche (2, 200, 300) disposée dans un logement (4) prévu dans le boîtier (3), cartouche dans laquelle est contenu un parfum (D1 ou D2), comprenant une ouverture d'entrée (5) par laquelle de l'air peut passer dans le boîtier (3) du dispositif (1) servant à parfumer, un espace de mélange (6) dans lequel le parfum (D1 et/ou D2) peut être mélangé avec l'air, une ouverture de sortie (7) par laquelle l'air mélangé avec le parfum (D1 et/ou D2) peut passer à l'extérieur, et au moins un dispositif de fermeture, pouvant être actionné, qui, dans au moins une position, empêche le parfum (D1 et/ou D2) de sortir du logement (4) et de passer dans l'espace de mélange (6) où, dans la zone de l'ouverture d'entrée (5) et/ou entre l'ouverture d'entrée (5) et l'espace de mélange (6), il est prévu un dispositif générateur de tourbillons (14), où le dispositif de fermeture est disposé entre le logement (4) et l'espace de mélange (6), et le dispositif de fermeture est formé par un volet (8),
**caractérisé en ce que** le logement (4), pour la cartouche est les cartouches (2, 200, 300), est configuré en forme de tiroir, et **en ce qu'**une ou plusieurs lèvres d'étanchéité (19) sont disposée sur le boîtier (3), lèvres d'étanchéité qui, pour assurer l'étanchéité, s'appliquent sur une surface de la cartouche (32, 232, 332), et le logement (4) pour la cartouche ou les cartouches (2, 200, 300) est disposé sur le côté de l'espace de mélange (6), faisant face au dispositif générateur de tourbillons (14), où l'axe de pivotement (9) du volet (8) s'étend parallèlement à la direction d'insertion du logement (4).

2. Dispositif servant à parfumeur selon la revendication 1, **caractérisé en ce que** le dispositif générateur de tourbillons (14) est forme par une roue à aubes rotative (13) et/ou par des aubes directrices fixes.

3. Dispositif servant à parfumer selon la revendication 1 ou 2, **caractérisé en ce que** le logement (4) est précontraint par un ressort.

4. Dispositif servant à parfumer selon l'une quelconque des revendications 1 à 3, **caractérisé en ce que** le dispositif de fermeture est formé par deux éléments mobiles l'un par rapport à l'autre et comportant des ouvertures, ou bien par un volet jalousie.

5. Dispositif servant à parfumer selon l'une quelconque des revendications précédentes, comprenant la cartouche (2, 200, 300) servant à la diffusion d'un parfum et présentant une partie fermant le fond (31, 231, 331), une partie couvrante (32, 232, 332) et des parois latérales (33, 233, 333) reliant la partie formant le fond et la partie couvrante, parois latérales qui entourent un espace dans lequel est placé un parfum ou un support diffuseur de parfum, ou il est prévu, dans la partie couvrante (32, 232, 332), une ou plusieurs ouvertures (34, 234, 334) à travers lesquelles le parfum peut se diffuser.

6. Dispositif servant à parfumer selon la revendication 5, **caractérisé en ce que** la totalité de la surface des ouvertures (34, 234, 334), dans la partie couvrante (32, 232, 332), est inférieure à 80 %.

7. Dispositif servant à parfumer selon la revendication 5 ou 6, **caractérisé en ce qu'**une lèvre d'étanchéité (30) est disposée sur la partie couvrante lèvre d'étanchéité qui s'étend sur la zone de bordure, de façon circulaire.

8. Dispositif servant à parfumer selon l'une quelconque des revendications 5 à 7, **caractérisé en ce que** deux parois latérales se faisant face (33, 233, 333) s'étendent de façon non exactement parallèle.

9. Dispositif servant à parfumer selon l'une quelconque des revendications 5 à 8, **caractérisé en ce qu'**au moins une paroi latérale (36, 236, 336) est configurée en étant amovible ou en prouvant pivoter sur la partie couvrante ou sur la partie formant le fond.

10. Dispositif servant à parfumeur selon l'une quelconque des revendications 5 à 9, **caractérisé en ce que** la cartouche (2, 200, 300), dans une position de stockage ou de transport, est formée au moyen d'un couvercle (241, 341) qui présente un bord (242, 342) en forme de crochet et entoure, sur la partie couvrante (232, 332), des parties saillantes correspondantes (243, 343).
